# EUROPEAN PATENT SPECIFICATION

(11) **EP 3 359 462 B1**
(45) Date of publication and mention of the grant of the patent: **09.12.2020**
(21) Application number: 16781525.7
(22) Date of filing: 28.09.2016
(51) Int. Cl.: B65D 51/28, B65D 81/32

(54) **DISPENSING CAPSULE**
AUSGABE VON KAPSELN
CAPSULE DE DISTRIBUTION

(30) Priority: 09.10.2015 GB 201517870
(43) Date of publication of application: 15.08.2018
(73) Proprietor: Tristel PLC, Snailwell, Newmarket CB8 7NY (GB)
(72) Inventor: TURNER, Jeremy, Omorokoa 3114 (NZ)
(74) Representative: Dummett Copp LLP
(86) International application number: PCT/GB2016/053021
(87) International publication number: WO 2017/060677

(56) References cited:
- EP-A1- 1 810 934
- US-A1- 2010 044 377
- US-B1- 6 644 471
- US-B1- 8 839 982

## Description

### BACKGROUND

### a. Field of the Invention

The present invention relates to a multi-chamber dispensing capsule, notably for dispensing a disinfectant composition.

### b. Related Art

Many liquid preparations include active ingredients which degrade over time, limiting product shelf life. This is particularly true for disinfectants or sterilising agents such as chlorine dioxide, where the active ingredient is formed in situ when required by mixing two reagents. Examples are disclosed in WO 2005/011756. Chlorine dioxide, for example, may be formed by mixing a chlorite solution and an acid.

It is known to provide a dispensing capsule for location in the neck of a vessel, the capsule having two internal chambers, each containing a reagent. Discharging of the contents of the chambers into the vessel allows the reagents to mix and generate the active ingredient. Examples of such a dispensing capsule are described in US 8,839,982. The dispensing capsule has two or more independently sealed and activated dispensing chambers each of which can contain a different substance to be dispensed into a primary chamber.

A problem with prior art dispensing capsule systems is that the capsule volume is typically small compared to the volume of the primary chamber. Accordingly, the reagents are present in a concentrated form and are dispensed into a diluent liquid such as water in the primary chamber. There may be a considerable delay between dispensing the contents of the chambers and generating an adequate concentration of active agent in the primary chamber. It is desirable to reduce or minimise this delay.

US 2010/0044377 discloses a device for mixing substances, whether in the form of a fixed or removable cap or in the form of emersion within a container, utilizing individual internal substance compartments, controlled by an external actuation mechanism.

EP 1 810 934 discloses a partitioned package structure for separately containing liquid material and solid material and allowing the liquid material and solid material to be conveniently mixed. The package structure includes a plastic seat directly recessed to form at least two separate receiving spaces. The liquid material and solid material are respectively contained in the receiving spaces. The receiving space containing the fluid material has a tapered flow way directed to the receiving space containing the solid material. A prior releasable joint section with poorer adhesive strength is disposed at a tip end of the flow way. When squeezing the receiving space containing the fluid material, the prior releasable joint section is broken to extrude the fluid material into the receiving space containing the solid material. The solid material is then soaked in and mixed with the fluid material. Then the cover membrane can be torn off to use the mixture.

US 6,644,471 discloses a capsule that is inserted into the neck of a bottle, or within a pull-up liquid dispenser cap, the capsule being a container or receptacle for sealably containing a liquid and/or a dry material and a dispenser for releasing the material when desired into the bottle through the orifice previously occupied by first and second plugs frangibly sealed in a first position and unsealed mechanically by the consumer depressing an elongated shaft releasing the liquid and/or dry materials into the container body in a second position.

### SUMMARY OF THE INVENTION

The invention is specified in the independent claims. Preferred features are specified in the dependent claims.

The invention provides for at least partial pre-mixing of the contents of the chambers before the mixture is discharged into the primary chamber. This pre-mixing accelerates formation of the active agent and reduces or minimises any delay in formation of an adequate concentration of active agent in the primary chamber.

### BRIEF DESCRIPTION OF THE DRAWINGS

The invention will now be further described, by way of example only, with reference to the following drawings in which:
Figures 1 and 2 show views of a partial assembly of dispensing capsule in accordance with an embodiment of the present invention;
Figure 3 is an x-ray view of the partial assembly of Figure 2;
Figure 4 is a central sectional view through the partial assembly of Figure 2;
Figures 5-7 show stages in the manufacture of a dispensing capsule in accordance with an embodiment of the present invention;
Figure 8 illustrates a partial assembly for manufacturing a dispensing capsule in accordance with another embodiment of the invention;
Figures 9-12 illustrate stages in the use of the dispensing capsule of Figure 7;
Figures 13-15 show a capsule body for use in alternative embodiments of the invention;
Figures 16-26 illustrate a cap assembly and stages in its use in an embodiment of the invention;
Figure 27 shows another embodiment of the capsule body ; and
Figure 28 shows the capsule body of Figure 27 in the neck of a vessel.

### DETAILED DESCRIPTION

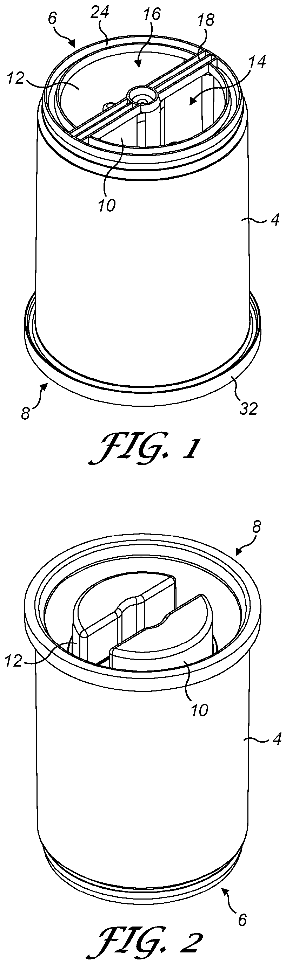

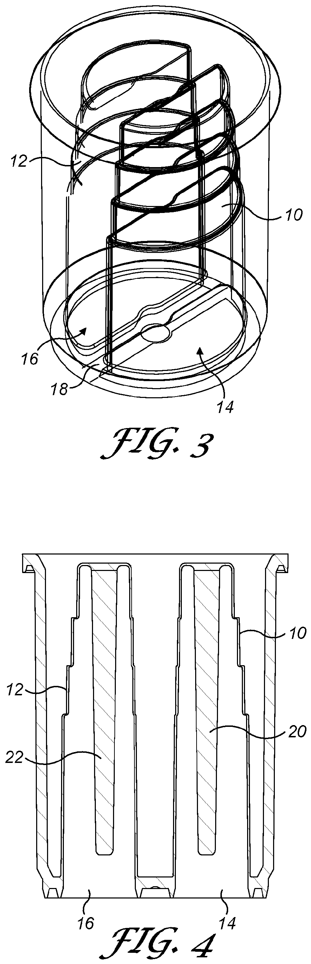

The partial assembly shown in Figures 1-4 comprises a capsule body 4 having a first end 6 and a second end 8. A first cavity 14 is defined within the capsule body 4 by at least a first wall 10, and a second cavity 16 is defined within the capsule body 4 by at least a second wall 12. A dividing member 18 is between the first cavity 14 and the second cavity 16. The dividing member 18 may be a common wall separating the cavities or it may be provided as an additional wall. The capsule body 4 has a peripheral wall 24 at the first end 6 and in this embodiment, a flange 32 at the second end. The cavities 14,16 each have a wall which is collapsible, concertina-style, as best shown in Figures 3 and 4. The first cavity 10 has a first burst pin 20 and the second cavity 12 has a second burst pin 22, as illustrated in Figure 4.

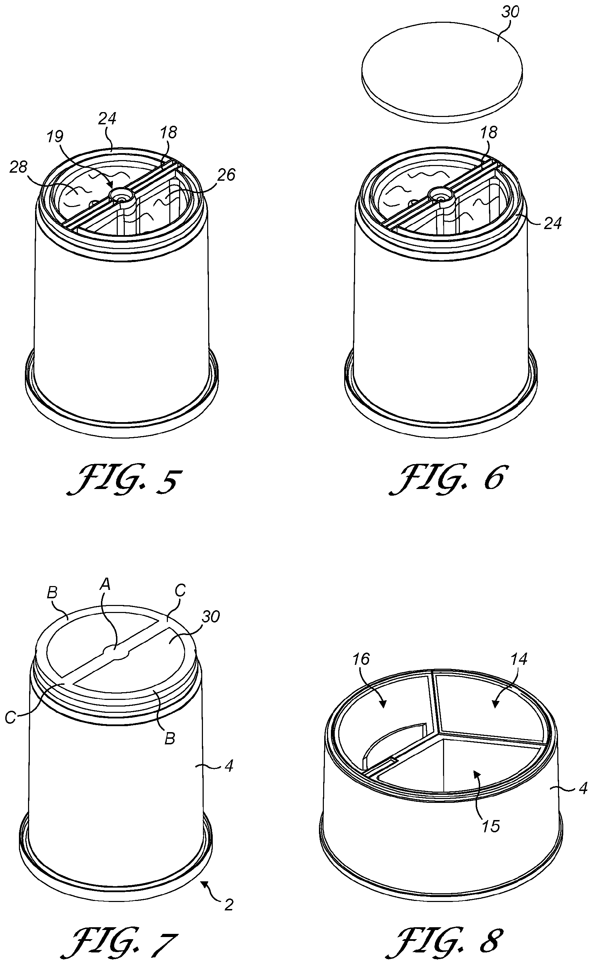

Referring now to Figures 5-7, stages in the manufacture of an exemplary dispensing capsule 2 are illustrated. The first cavity 14 is at least partly filled with a first fluid containing a first reagent 26, and the second cavity 16 is at least partly filled with a second fluid containing a second reagent 28. The term "fluid" is used herein to include liquids, pastes, aerosols, powders, sols and gels. The dividing member 18 in this embodiment has a depression or hollow region 19 corresponding to an injection point in the manufacture of the capsule body 4. A flexible seal 30 is bonded to the peripheral wall 24 at the first end of the capsule body 4, and to the dividing member 18 so as to seal the contents of the first cavity 14 from the contents of the second cavity 16. In this example, the seal 30 is welded to the peripheral wall 18 by an outer weld B and a retention weld region C-C (Figure 7). A central weld region A is particularly weakly bonded because of the depression 19 where the seal 30 makes less contact with the underlying dividing member 18.

The seal 30 is plastically or elastically deformable under pressure. Suitable seal materials will be known to those skilled in the art. The seal 30 may, for example, be formed from a laminate of polyethylene (PE)/polyamide (PA)/ethylene-vinyl acetate (EVA) or PE/Aluminium/EVA. The capsule body 4 may be formed from any suitable structural material, notably a plastics material such as LDPE. The seal 30 may be bonded to the peripheral wall 24 and dividing member 18 by any suitable techniques; for example by welding or by means of an adhesive. Suitable adhesives and welding techniques will be well known to those skilled in the art.

It will be appreciated that the capsule body 4 may be of any size appropriate to its intended use, and may include more than two chambers. For example, the capsule body 4 shown in Figure 8 is wider and shorter than the capsule body 4 of Figure 1, and it includes a third cavity 15 in addition to the first cavity 14 and second cavity 16. The third cavity 15 may include a third fluid with a third reagent.

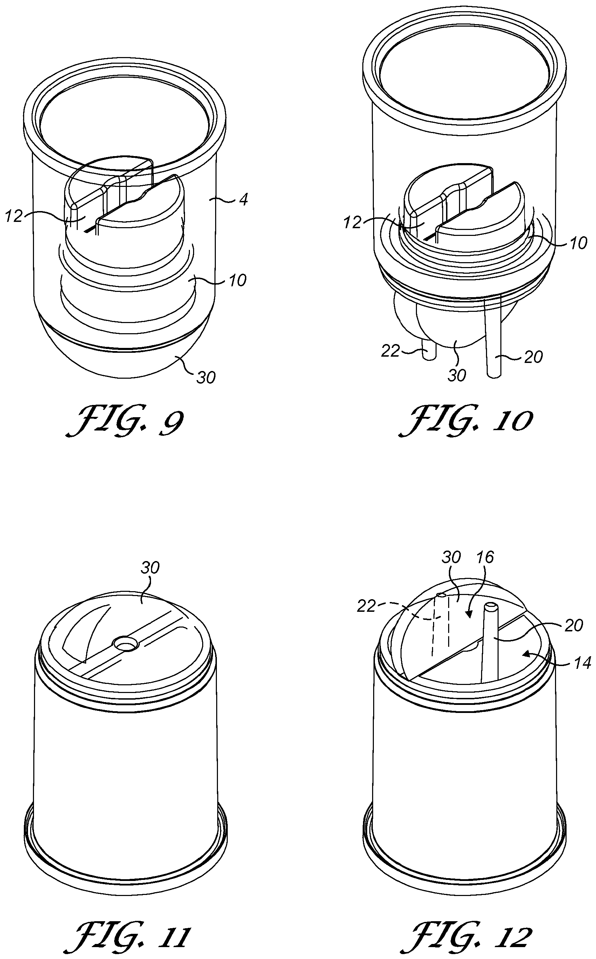

Referring now to Figures 9-12, stages in the use of the dispensing capsule 2 are illustrated. Application of pressure to the walls 10,12 of the cavities 14,16 causes the walls to progressively collapse concertina-style. If the pressure within the cavities is progressively increased, a critical pressure is reached at which the bond between the seal 30 and the dividing member 18 breaks, initially at the depression 19 which is the weakest point, allowing the seal 30 to be deformed into a dome (Figures 9 and 11). This deformation permits the contents of the first cavity 14 to at least partly mix with the contents of the second cavity 16 while the bond between the seal 30 and the peripheral wall 24 remains intact. The contents may, for example, be reagents which when mixed produce a disinfectant composition; for example chlorine dioxide or peracetic acid. Suitable reagents will be well known to those skilled in the art; for example, reagents for producing chlorine dioxide include: chlorite and acid; chlorate, peroxide and acid; and chlorite, hypochlorite, and a suitable buffer. The reagents may be in a concentrated form, providing rapid formation of the active agent when the contents of the chambers are mixed.

Increasing the applied pressure further collapses the walls 10,12 of the cavities 14,16, bringing the tips of the burst pins 20,22 into contact with the seal 30 and then pushing against the seal 30 so as to break the bond at the outer weld B (Figures 10 and 12). This arrangement permits the pre-mixed contents of the capsule body 4 to be dispensed in a controlled manner.

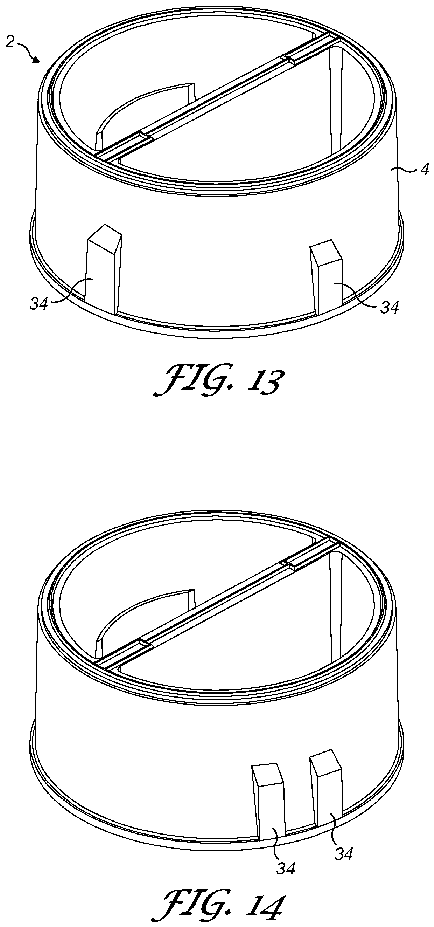

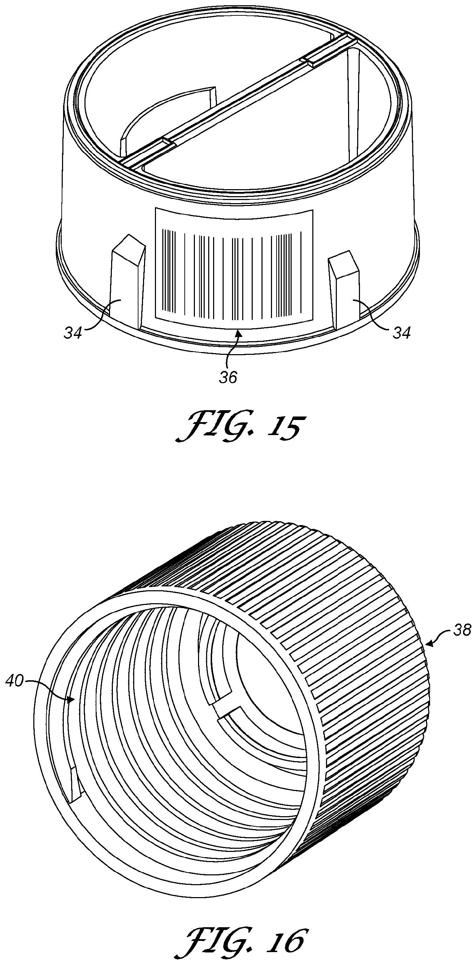

As illustrated in the embodiments of Figures 13-15, ribs 34 may be provided on the side of the capsule body 4. The ribs 34 act as a mechanical 'fingerprint' to identify the type or dose size carried by the dispensing capsule. The ribs 34 restrict or prevent the use of incorrect dispensing capsules by not permitting them to be engaged in a receiving portion of a vessel. The ribs 34 may also be used to mechanically activate the vessel to alert it to the type of capsule being used and what dose and vessel volume are to be filled. The ribs 34 may also be used to locate a bar code 36 (Figure 15) that can be scanned by the vessel during the dispensing process. The bar code can also deliver information about the dispensing capsule for traceability and regulatory procedures.

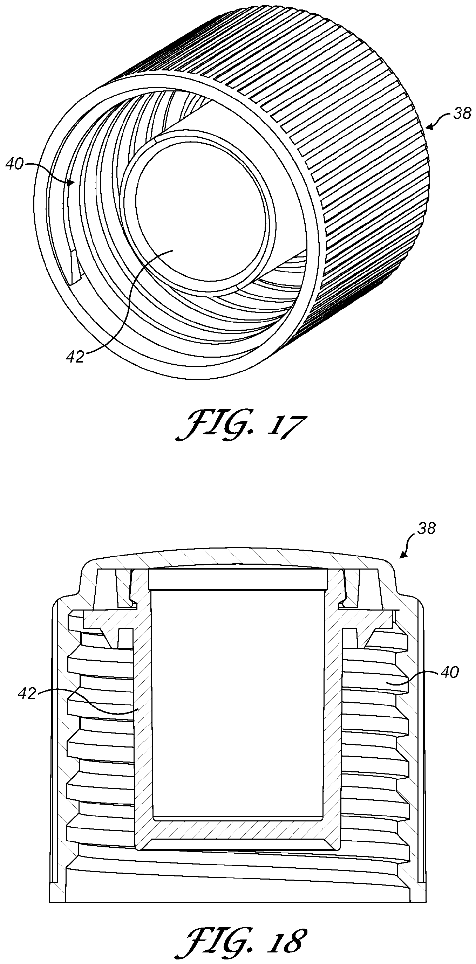

To apply force to the second (crush) end of the capsule body, a dedicated screw cap 38 (Figures 16-18) may be provided for use with the vessel. The cap 38 has an internal screw thread 40. Inside, a plunger 42 is attached which is able to rotate.

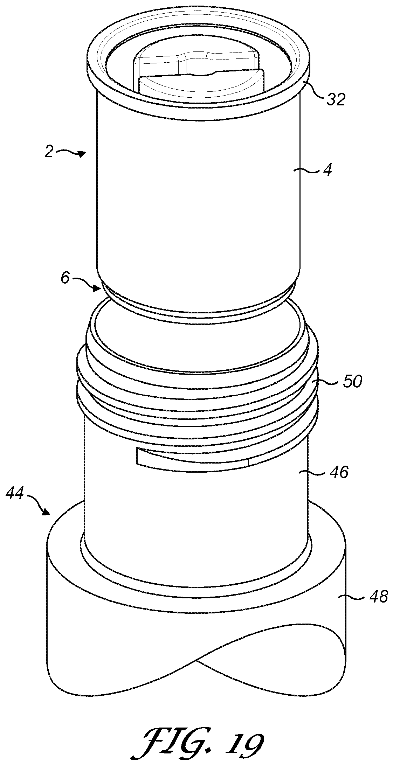

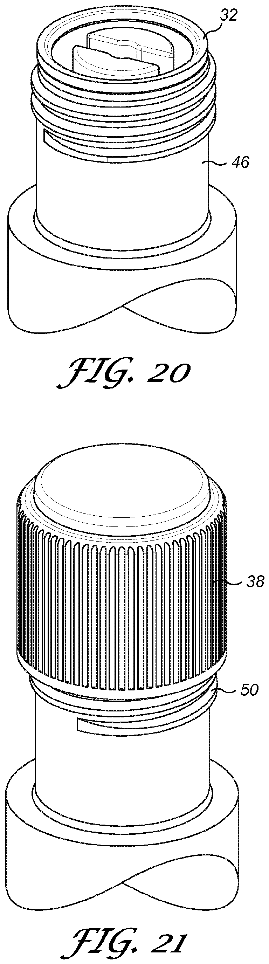

An implementation of the dispensing capsule in a vessel 44 is illustrated in Figures 19-26. The vessel 44 in this example is a bottle which has a neck 46 and a primary chamber 48. The neck 46 is provided with an external screw thread 50 which is complementary to the internal screw thread 40 on the cap 38. The capsule 2 is inserted into the neck 46, with the first (burst) end 6 innermost so that the seal 30 is in fluid communication with the primary chamber 48. The capsule body 4 is sized to be a close fit in the neck 46. When the capsule 2 is fully inserted, the flange 32 on the capsule body 4 sits on the rim of the neck 46 and a seal is created between the capsule 2 and the neck 46.

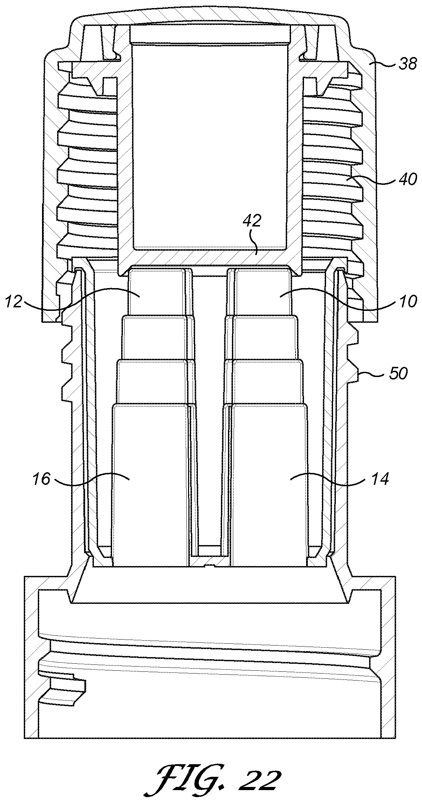

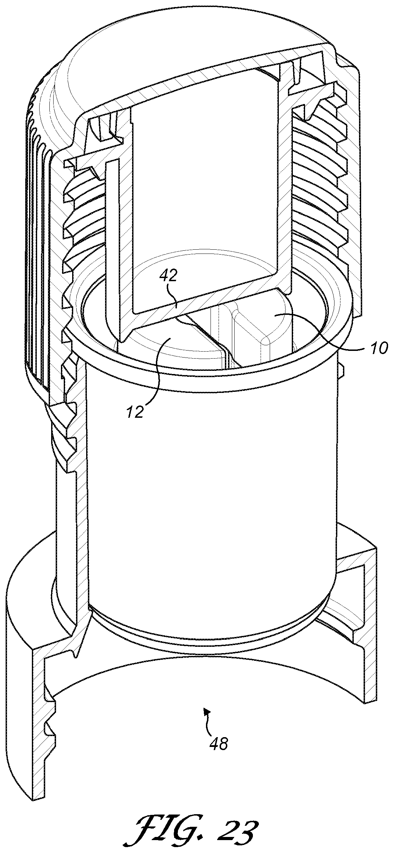

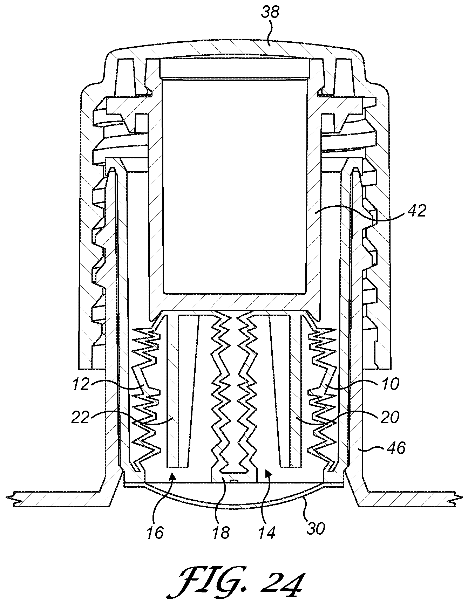

The cap 38 is then placed over the neck 46 of the vessel 44 and turned to engage the screw threads 40,50 with each other. When the cap 38 has been turned sufficiently, the plunger 42 is brought into engagement with walls 10,12 of the cavities 14,16 (Figures 22 and 23). Further turning of the cap 38 by the user advances the plunger 42 towards the first end of the capsule 2 and causes controlled collapsing of the walls 10,12 of the cavities 14,16. The collapsing of the walls increases pressure within the cavities 14,16. When a critical pressure is reached, the bond between the seal 30 and the dividing member 18 is broken (Figure 24) allowing the contents of the first chamber 14 and second chamber 16 at least partly to mix while being retained within the peripheral boundary of the seal 30.

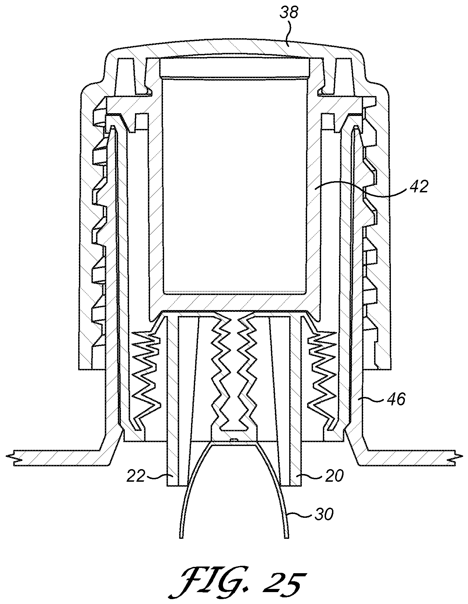

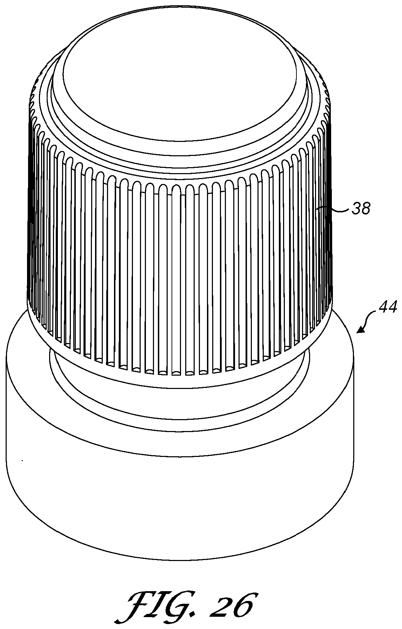

Still further turning of the cap 38 (Figures 25 and 26) advances the plunger 42 farther and causes the burst pins 20,22 to break the bond between the seal 30 and at least some of the peripheral wall 24, allowing the mixed contents of the capsule 2 to be dispensed into the primary chamber 48 of the vessel 44. Dispensing may be further facilitated by further turning of the cap 38 driving the plunger 42 down to displace the cavity area and force the mixture out of the capsule.

The primary chamber 48 contains a diluent liquid, for example water, into which the premixed concentrate from the capsule is dispensed. Because the concentrated reagents are at least partly mixed together before dilution, formation of the active agent is accelerated, thereby reducing or minimising any delay in formation of an adequate concentration of active agent in the primary chamber.

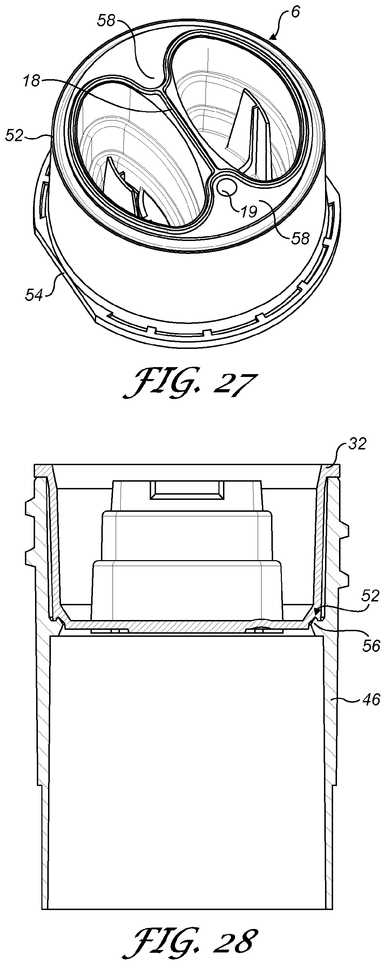

Referring now to Figures 27 and 28, a further embodiment of a capsule body for use in the invention is shown. Here, the dividing member 18 is disposed between two internal projections 58 at the second end 6. The depression 19 is located on one of the projections 58. The capsule body has a production key detail 54 which is used to locate the capsule during filling and sealing operations. A sealing groove 52 is provided at the second end 6. The neck 46 of a vessel has an internal flange 56 onto which the sealing groove 52 locates when the capsule is inserted in the neck 46 of a vessel. The arrangement provides for improved sealing of the contents of the vessel both before and after dispensing of the contents of the capsule.

### Experimental

Prototype capsule bodies similar to those shown in Figures 1-7 were formed from LDPE. Each capsule body was 35 mm deep and had a 26 mm external diameter at the first end and a 31 mm external diameter at the second end. The capsule outer wall was 1.2 mm thick. The internal walls defining the cavities were 0.5 mm thick and the cavities had a depth of 34 mm. Each cavity was filled with 3.5 ml of water and then sealed. The laminate seal was formed from PE (30 µm)/PA (30 µm)/EVA (40 µm) and was bonded to the first end by a hot-weld machine set at 135°C, for 3 seconds. Test results are given below.

### 1.0 CRUSH FORCE

### 1.1 Test Objectives

To determine that the proposed crush cycle operation is working correctly to first pre-mix the contents of the two cavities prior to being released from the capsule.

To determine the forces required to achieve this crush cycle and how these forces vary at various stages of this cycle.

To determine the effect that temperature may have on these forces.

### 1.2 Test Method

a) A loaded capsule is fitted into a test neck jig with the cap plunger placed on top surface. The press is then centrally positioned. The scales read the force applied by the press onto the test assembly.
b) The force is recorded at three positions through the crush cycle
c) The first 5 mm of plunger travel prior to any weld burst, both liquid filled cavities are still independently sealed. The seal is starting to bulge under the pressure applied
d) The Pre Burst position is when the central weld has broken to allow the liquid contents of both cavities to mix - the seal has its maximum size bulge with the perimeter weld still fully attached
e) The Release has the seal opened, at both cavities, from the burst pins driving through the seal during the final stage of the crush cycle - the seal has broken away from the weld to release the contents
f) Samples were tested at various temperatures

### 1.3 Test Results

Averages from test results are given in Table 1.

**TABLE 1**

| °C | Kg | Kg | Kg |
|---|---|---|---|
| capsule Temp. | 5mm | Pre Burst | Release |
| | | | |
| 34 | 5 | 5.8 | 9 |
| 22 | 6 | 11 | 14 |
| 23 | 6 | 12 | 15 |
| 14 | 7 | 15 | 20 |
| 10 | 12 | 20 | 22 |

### 1.4 Summary

The results show that increased forces are required to complete the crush cycle when the capsule has a lower temperature. A limit may need to be placed on the temperature range to allow for correct operation of the capsule.

The capsule performed as expected completing the required weld burst function at each of the three stages of the cycle. The Pre Burst stage showed a clear detachment of the central weld that allowed the contents of the two cavities to fully mix prior to being released out of the capsule.

### 1.5 Conclusions

Temperature has an effect on the forces required to operate the capsule.

The forces required increase as the temperature lowers.

The capsule pre burst and release cycle is performing as required.

### 2.0 CRUSH PRESSURE

### 2.1 Test Objectives

To determine the amount of pressure that is being generated within the capsule during the crush cycle at the various stages. These results can then be used to help specify the limits of the weld retention.

### 2.2 Test Method

a) An empty capsule is fitted into the test jig with the cap plunger assembled to the top surface. A press is applied to the test assembly. The pressure gauge is assembled to record the resulting pressure change.
b) The pressure is recorded at three positions through the crush cycle: 5 mm; Pre Burst; Release.

### 2.3 Test Results

2.4 Results are given in Table 2. The final column records that the finished Pre-Burst pressure held for 30 seconds, confirming that no leakage is occurring.

**TABLE 2**

| Pa | Pa | Pa | 30 seconds |
|---|---|---|---|
| 5mm | Pre Burst | Release | Pressure |
| 2758 | 4137 | 5516 | held |
| 1379 | 3447 | 5516 | held |
| 1379 | 3447 | 5516 | held |
| 1379 | 3447 | 6205 | held |
| 1379 | 3447 | 5516 | held |
| 1379 | 3447 | 5516 | held |
| 1379 | 3447 | 5516 | held |
| 1379 | 3447 | 5516 | held |

### 2.5 Summary

The pressure levels shown are of a lower range which is easier to control. A pattern is evident from the results that suggest consistency has occurred. Weld strength can now be based on these pressures to determine the best burst results.

### 2.6 Conclusion

The consistency suggests the capsule is not leaking.

The crush effect produces a consistent pressure result through the cycle.

### 3.0 SCREW CAP TORQUE

### 3.1 Test Objectives

To determine the torque levels being applied to the cap during the crush cycle at the various stages. The cap has to be user friendly so that the cap is easily screwed on during standard operation. Excess torque forces would require changes to the design to bring the forces to acceptable levels.

### 3.2 Test Method

a) A loaded capsule is fitted into a test neck. The cap with plunger assembled is threaded onto the neck. A torque meter is attached to the top of the cap. Recordings are taken at the three positions.
b) The lower torque levels recorded are based on best readings using the torque meter used.
c) The cap has a thread set at 5.5 turns per 20 mm for these tests.
d) The pressure is recorded at three positions through the crush cycle (described in 2.2 above): 5 mm; Pre Burst; Release.

### 3.3 Test Results are given in Table 3.

**TABLE 3**

| Nm | Nm | Nm |
|---|---|---|
| 5 mm | Pre Burst | Release |
| 0.2 | 0.5 | 1.8 |
| 0.3 | 0.4 | 1.2 |
| 0.3 | 0.4 | 1.2 |
| 0.3 | 0.5 | 1.5 |
| 0.3 | 0.6 | 1.5 |
| 0.3 | 0.4 | 1.2 |
| 0.3 | 0.4 | 1.2 |
| 0.3 | 0.5 | 1.4 |
| 0.3 | 0.4 | 1.8 |
| 0.3 | 0.5 | 1.4 |
| 0.3 | 0.4 | 1.6 |
| 0.3 | 0.4 | 1.2 |
| 0.3 | 0.5 | 1.6 |
| 0.3 | 0.4 | 1.4 |
| 0.3 | 0.5 | 1.4 |

### 3.4 Conclusion

The current torque levels are well within an industry standard for comfortable ergonomic operation of the screw cap.

There is room to change the screw cap thread to reduce the number of turns required to complete a crush cycle.

## Claims

1. A dispensing capsule (2) comprising a capsule body (4) having a first end (6) and a second end (8);
at least one collapsible wall (10) defining a first cavity (14) within the capsule body (4), the first cavity (14) having an opening at the first end (6) of the capsule body (4);
at least one collapsible wall (12) defining a second cavity (16) within the capsule body (4), the second cavity (16) having an opening at the first end (6) of the capsule body (4);
a dividing member (18) between the first cavity (14) and the second cavity (16);
a peripheral wall (24) at the first end (6) of the capsule body (4); and
a flexible seal (30) bonded to the peripheral wall (24) at the first end (6) of the capsule body (4) and to the dividing member (18) so as to seal the contents of the first cavity (14) from the contents of the second cavity (16);
wherein the seal (30) is bonded less strongly to the dividing member (18) than to the peripheral wall (24);
further comprising a burst pin (20,22) disposed within each of the first cavity (14) and the second cavity (16), each burst pin (20,22) having a tip;
wherein application of pressure to the walls (10, 12) of the cavities (14, 16) causes the walls to progressively collapse, progressively increasing the pressure within the cavities (14, 16) to a critical pressure at which the bond between the seal (30) and the dividing member (18) will break, permitting the contents of the first cavity (14) to at least partly mix with the contents of the second cavity (16) while the bond between the seal (30) and the peripheral wall (24) remains intact; and wherein further collapsing of the walls (10, 12) of the cavities (14, 16) will cause the tip of each burst pin (20,22) to press against the seal (30) and cause breaking of the bond between the seal (30) and at least some of the peripheral wall (24) as pressure within the first cavity (14) and the second cavity (16) is progressively increased beyond the critical pressure.

2. A dispensing capsule according to claim 1, wherein a region of the dividing member (18) to which the seal (30) is bonded has a depression or hole (19) which reduces the area of contact between the dividing member (18) and the seal (30).

3. A dispensing capsule according to claim 2, wherein the dividing member (18) is disposed between two internal projections (58) at the first end (6), the depression or hole (19) located on one of the projections (58).

4. A dispensing capsule according to any one of the preceding claims, wherein the walls (10, 12) of the first cavity (14) and the second cavity (16) are each collapsible concertina-style if pressure is applied to the walls (10, 12) from the second end (8) of the capsule body (4).

5. A dispensing capsule according to any one of the preceding claims, wherein the first cavity (14) contains a first reagent and the second cavity (16) contains a second reagent, and wherein the first reagent and the second reagent react when mixed to produce a disinfectant or sterilising agent.

6. A dispensing capsule according to any one of the preceding claims, wherein the capsule body (4) is provided with at least two external ribs (34).

7. An assembly for dispensing liquids, the assembly comprising a vessel (44) having a primary chamber (48) and a neck (46) in which is disposed a dispensing capsule (2) according to any one of the preceding claims; the seal (30) in fluid communication with the primary chamber (48).

8. An assembly according to claim 7, wherein the neck (46) includes an external screw thread (50); the vessel (44) further including a cap (38) having a complementary screw thread (40) and a plunger (42) which is disposed within the neck (46) when the cap (38) is fully engaged with the neck (46);
the arrangement being such that if the cap (38) is screwed sufficiently far onto the neck (46), the plunger (42) will initially be brought into contact with the walls of the first cavity (14) and the second cavity (16) through the second end (8) of the capsule body (4), and will then progressively crush the walls of the first cavity (14) and the second cavity (16) until the critical pressure is reached.

9. An assembly according to claim 8, wherein further screwing of the cap (38) onto the neck (46) after the critical pressure has been reached will cause the plunger (38) to further crush the walls of the cavities and cause each burst pin (20, 22) to break the bond between the seal (30) and at least some of the peripheral wall (24).

10. An assembly according to any one of claims 7 to 9, wherein the capsule body (4) and the neck (46) are each provided with at least one interengageable feature (52, 56).

11. An assembly according to claim 10, wherein the capsule body (4) is provided with two external ribs (34) and the neck (46) is provided with corresponding grooves for receiving the ribs.

12. An assembly according to claim 11, wherein the capsule body (4) further comprises a bar code (36) located between said two external ribs (34), the bar code (36) encoding information about the dispensing capsule (2).

13. An assembly according to claim 12, further comprising a bar code scanner for reading information encoded by the bar code (36).

14. An assembly according to any one of claims 7-13, wherein the capsule body (4) is provided with an external sealing groove (52) at the second end (8) and wherein the neck (46) of the vessel (44) is provided with an internal flange (56) onto which the sealing groove (52) locates.

## Patentansprüche

1. Ausgabekapsel (2), umfassend:
einen Kapselkörper (4) mit einem ersten Ende (6) und einem zweiten Ende (8);
wenigstens eine kollabierbare Wand (10), welche einen ersten Hohlraum (14) innerhalb des Kapselkörpers (4) definiert, wobei der erste Hohlraum (14) eine Öffnung am ersten Ende (6) des Kapselkörpers (4) aufweist;
wenigstens eine kollabierbare Wand (12), welche einen zweiten Hohlraum (16) innerhalb des Kapselkörpers (4) definiert, wobei der zweite Hohlraum (16) eine Öffnung am ersten Ende (6) des Kapselkörpers (4) aufweist;
ein Trennelement (18) zwischen dem ersten Hohlraum (14) und dem zweiten Hohlraum (16);
eine Umfangswand (24) am ersten Ende (6) des Kapselkörpers (4); und
eine flexible Dichtung (30), die mit der Umfangswand (24) am ersten Ende (6) des Kapselkörpers (4) und dem Trennelement (18) verbunden ist, um den Inhalt des ersten Hohlraums (14) vom Inhalt des zweiten Hohlraums (16) abzukapseln;
wobei die Dichtung (30) weniger fest mit dem Trennelement (18) verbunden ist als mit der Umfangswand (24) ;
wobei weiter sowohl innerhalb des ersten Hohlraums (14) als auch innerhalb des zweiten Hohlraums (16) ein Aufreißstift (20, 22) umfasst ist, wobei jeder Aufreißstift (20, 22) eine Spitze aufweist;
wobei das Ausüben von Druck auf die Wände (10, 12) der Hohlräume (14, 16) bewirkt, dass die Wände fortschreitend kollabieren, wodurch der Druck innerhalb der Hohlräume (14, 16) fortschreitend bis zu einem kritischen Druck ansteigt, bei welchem die Verbindung zwischen der Dichtung (30) und dem Trennelement (18) aufbricht, wodurch ermöglicht wird, dass sich die Inhalte des ersten Hohlraums (14) wenigstens teilweise mit den Inhalten des zweiten Hohlraums (16) mischen, während die Verbindung zwischen der Dichtung und der Umfangswand (24) intakt bleibt; und
wobei ein weiteres Kollabieren der Wände (10, 12) der Hohlräume (14, 16) bewirken wird, dass die Spitze jedes Aufreißstiftes (20, 22) gegen die Dichtung (30) drückt, und bewirken wird, dass die Verbindung zwischen der Dichtung (30) und wenigstens einem Teil der Umfangswand (24) aufbricht, während der Druck innerhalb des ersten Hohlraums (14) und des zweiten Hohlraums (16) fortschreitend über den kritischen Druck hinaus erhöht wird.

2. Ausgabekapsel nach Anspruch 1,
bei welcher ein Bereich des Trennelements (18), mit dem die Dichtung (30) verbunden ist, eine Vertiefung oder ein Loch (19) aufweist, welche(s) die Kontaktfläche zwischen Trennelement (18) und Dichtung (30) reduziert.

3. Ausgabekapsel nach Anspruch 2,
bei welcher das Trennelement (18) zwischen zwei inneren Vorsprüngen (58) am ersten Ende (6) angeordnet ist, wobei die Vertiefung oder das Loch (19) an einem der Vorsprünge (58) angeordnet ist.

4. Ausgabekapsel nach einem der vorigen Ansprüche,
bei welcher die Wände (10, 12) des ersten Hohlraums (14) und des zweiten Hohlraums (16) jeweils zieharmonikaartig kollabierbar sind, wenn ein Druck auf die Wände (10, 12) vom zweiten Ende (8) des Kapselkörpers (4) aus ausgeübt wird.

5. Ausgabekapsel nach einem der vorigen Ansprüche,
bei welcher der erste Hohlraum (14) eine erste Reagenz enthält und der zweite Hohlraum (16) eine zweite Reagenz enthält, und wobei die erste Reagenz und die zweite Reagenz reagieren, wenn sie miteinander gemischt werden, um ein Desinfektionsmittel oder Sterilisationsmittel herzustellen.

6. Ausgabekapsel nach einem der vorigen Ansprüche,
bei welcher der Kapselkörper (4) mit wenigstens zwei externen Rippen (34) versehen ist.

7. Anordnung zum Verspenden von Flüssigkeiten, wobei die Anordnung ein Gefäß (44) mit einer Hauptkammer (48) und einem Hals (46) umfasst, worin eine Ausgabekapsel (2) nach einem der vorigen Ansprüche angeordnet ist; wobei sich die Dichtung (30) in Fluidverbindung mit der Hauptkammer (48) befindet.

8. Anordnung nach Anspruch 7,
bei welcher der Hals (46) ein Außengewinde (50) aufweist; wobei das Gefäß (44) weiter eine Kappe (38) mit einem Gegengewinde (40) und einem Druckstück (42) um-fasst, welches innerhalb des Halses (46) angeordnet ist, wenn die Kappe (38) vollständig mit dem Hals (46) verbunden ist;
wobei die Anordnung derart ausgebildet ist, dass wenn die Kappe (38) hinreichend weit auf den Hals (46) aufgeschraubt wird, das Druckstück (42) zu Anfang in Kontakt mit den Wänden des ersten Hohlraums (14) und des zweiten Hohlraums (16) durch das zweite Ende (8) des Kapselkörpers (4) gebracht wird und anschließend fortschreitend die Wände des ersten Hohlraums (14) und des zweiten Hohlraums (16) eindrückt, bis der kritische Druck erreicht ist.

9. Anordnung nach Anspruch 8,
bei welcher das Weiterschrauben der Kappe (38) auf den Hals (46) nach Erreichen des kritischen Drucks bewirkt, dass das Druckstück (38) die Wände der Hohlräume weiter eindrückt und bewirkt, dass jeder Aufreißstift (20, 22) die Verbindung zwischen der Dichtung (30) und wenigstens einem Teil der Umfangswand (24) aufbricht.

10. Anordnung nach einem der Ansprüche 7 - 9,
bei welcher der Kapselkörper (4) und der Hals (46) jeweils mit wenigstens einer austauschbaren Einrichtung (52, 56) versehen sind.

11. Anordnung nach Anspruch 10,
bei welcher der Kapselkörper (4) mit zwei externen Rippen (34) und der Hals (46) mit entsprechenden Rillen zur Aufnahme der Rippen versehen ist.

12. Anordnung nach Anspruch 11,
bei welcher der Kapselkörper (4) weiter einen Barcode (36) umfasst, der sich zwischen den zwei externen Rippen (34) befindet, wobei der Barcode (36) Informationen über die Ausgabekapsel (2) in codierter Form aufweist.

13. Anordnung nach Anspruch 12,
weiter umfassend einen Barcodescanner zum Auslesen der durch den Barcode (36) codierten Informationen.

14. Anordnung nach einem der Ansprüche 7 - 13,
bei welcher der Kapselkörper (4) mit einer externen Dichtrille (52) am zweiten Ende (8) versehen ist und bei welcher der Hals (46) des Gefäßes (44) mit einem Innenflansch (56) versehen ist, auf welchem sich die Dichtrille (52) anordnen lässt.

## Revendications

1. Capsule de distribution (2) comprenant un corps de capsule (4) ayant une première extrémité (6) et une seconde extrémité (8) ;
au moins une paroi pliable (10) définissant une première cavité (14) à l'intérieur du corps de capsule (4), la première cavité (14) ayant une ouverture à la première extrémité (6) du corps de capsule (4) ;
au moins une paroi pliable (12) définissant une seconde cavité (16) à l'intérieur du corps de capsule (4), la seconde cavité (16) ayant une ouverture à la première extrémité (6) du corps de capsule (4) ;
un élément de séparation (18) entre la première cavité (14) et la seconde cavité (16) ;
une paroi périphérique (24) à la première extrémité (6) du corps de capsule (4) ; et
un joint souple (30) lié à la paroi périphérique (24) au niveau de la première extrémité (6) du corps de capsule (4) et à l'élément de séparation (18) de façon à sceller de manière étanche le contenu de la première cavité (14) vis-à-vis du contenu de la seconde cavité (16) ;
le joint (30) étant moins fortement lié à l'élément de séparation (18) qu'à la paroi périphérique (24) ;
comprenant en outre une tige d'éclatement (20, 22) disposée à l'intérieur de chacune de la première cavité (14) et de la seconde cavité (16), chaque tige d'éclatement (20, 22) ayant une pointe ;
l'application d'une pression sur les parois (10, 12) des cavités (14, 16) amenant les parois à se plier progressivement, augmentant progressivement la pression à l'intérieur des cavités (14, 16) jusqu'à une pression critique à laquelle la liaison entre le joint (30) et l'élément de séparation (18) rompra, permettant au contenu de la première cavité (14) de se mélanger au moins partiellement avec le contenu de la seconde cavité (16) tandis que la liaison entre le joint (30) et la paroi périphérique (24) reste intact ; et un pliage supplémentaire des parois (10, 12) des cavités (14, 16) amenant la pointe de chaque tige d'éclatement (20, 22) à appuyer contre le joint (30) et entraînant une rupture de la liaison entre le joint (30) et au moins une partie de la paroi périphérique (24) à mesure que la pression dans la première cavité (14) et la seconde cavité (16) est progressivement augmentée au-delà de la pression critique.

2. Capsule de distribution selon la revendication 1, dans laquelle une région de l'élément de séparation (18) à laquelle le joint (30) est lié a un creux ou trou (19) qui réduit la surface de contact entre l'élément de séparation (18) et le joint (30).

3. Capsule de distribution selon la revendication 2, dans laquelle l'élément de séparation (18) est disposé entre deux saillies internes (58) à la première extrémité (6), le creux ou trou (19) étant situé sur l'une des saillies (58).

4. Capsule de distribution selon l'une quelconque des revendications précédentes, dans laquelle les parois (10, 12) de la première cavité (14) et de la seconde cavité (16) sont chacune pliables en accordéon si une pression est appliquée sur les parois (10, 12) à partir de la seconde extrémité (8) du corps de capsule (4).

5. Capsule de distribution selon l'une quelconque des revendications précédentes, dans laquelle la première cavité (14) contient un premier réactif et la seconde cavité (16) contient un second réactif, et le premier réactif et le second réactif réagissant lorsqu'ils sont mélangés pour produire un agent désinfectant ou stérilisant.

6. Capsule de distribution selon l'une quelconque des revendications précédentes, dans laquelle le corps de capsule (4) comporte au moins deux nervures externes (34).

7. Ensemble pour distribuer des liquides, l'ensemble comprenant un récipient (44) ayant une chambre primaire (48) et un goulot (46) dans lequel est disposée une capsule de distribution (2) selon l'une quelconque des revendications précédentes ; le joint (30) étant en communication fluidique avec la chambre primaire (48).

8. Ensemble selon la revendication 7, dans lequel le goulot (46) comprend un filetage externe (50) ; le récipient (44) comprenant en outre un capuchon (38) ayant un filetage complémentaire (40) et un poussoir (42) qui est disposé à l'intérieur du goulot (46) lorsque le capuchon (38) est entièrement engagé avec le goulot (46) ;
l'agencement étant tel que si le capuchon (38) est vissé suffisamment loin sur le goulot (46), le poussoir (42) sera initialement amené en contact avec les parois de la première cavité (14) et de la seconde cavité (16) par l'intermédiaire de la seconde extrémité (8) du corps de capsule (4), puis écrasera progressivement les parois de la première cavité (14) et de la seconde cavité (16) jusqu'à ce que la pression critique soit atteinte.

9. Ensemble selon la revendication 8, dans lequel un vissage supplémentaire du capuchon (38) sur le goulot (46) une fois que la pression critique a été atteinte amènera le poussoir (38) à écraser davantage les parois des cavités et amènera chaque tige d'éclatement (20, 22) à rompre la liaison entre le joint (30) et au moins une partie de la paroi périphérique (24).

10. Ensemble selon l'une quelconque des revendications 7 à 9, dans lequel le corps de capsule (4) et le goulot (46) comportent chacun au moins un élément à engagement mutuel (52, 56).

11. Ensemble selon la revendication 10, dans lequel le corps de capsule (4) comporte deux nervures externes (34) et le goulot (46) comporte des rainures correspondantes pour recevoir les nervures.

12. Ensemble selon la revendication 11, dans lequel le corps de capsule (4) comprend en outre un code à barres (36) situé entre lesdites deux nervures externes (34), le code à barres (36) codant des informations concernant la capsule de distribution (2).

13. Ensemble selon la revendication 12, comprenant en outre un dispositif de balayage de code à barres pour lire des informations codées par le code à barres (36).

14. Ensemble selon l'une quelconque des revendications 7 à 13, dans lequel le corps de capsule (4) comporte une rainure d'étanchéité externe (52) à la seconde extrémité (8) et le goulot (46) du récipient (44) comporte une bride interne (56) sur laquelle se positionne la rainure d'étanchéité (52).
